# EUROPÄISCHE PATENTSCHRIFT

(11) **EP 3 148 464 B1**
(45) Veröffentlichungstag und Bekanntmachung des Hinweises auf die Patenterteilung: **03.03.2021**
(21) Anmeldenummer: 15729767.2
(22) Anmeldetag: 28.05.2015
(51) Int. Cl.: A61B 18/04

(54) **VORRICHTUNG ZUR BIOLOGISCHEN DEKONTAMINATION VON PERKUTANEN ZUGÄNGEN UND VERFAHREN HIERZU**
DEVICE FOR BIOLOGICALLY DECONTAMINATING PERCUTANEOUS ACCESS POINTS AND METHOD THEREFOR
DISPOSITIF POUR LA DÉCONTAMINATION BIOLOGIQUE D'ACCÈS PERCUTANÉS ET PROCÉDÉ ASSOCIÉ

(30) Priorität: 28.05.2014 DE 102014107554
(43) Veröffentlichungstag der Anmeldung: 05.04.2017
(73) Patentinhaber: Leibniz-Institut für Plasmaforschung und Technologie e.V., 17489 Greifswald (DE)
(72) Erfinder: WELTMANN, Klaus-Dieter, 18609 Binz (DE); STIEBER, Manfred, 17489 Greifswald (DE); KRAFCZYK, Stephan, 17489 Greifswald (DE); VON WOEDTKE, Thomas, 18519 Gerdeswalde (DE)
(74) Vertreter: Schulz Junghans Patentanwälte PartGmbB
(86) Internationale Anmeldenummer: PCT/EP2015/061900
(87) Internationale Veröffentlichungsnummer: WO 2015/181325

(56) Entgegenhaltungen:
- WO-A2-2006/116252
- DE-U1-202009 011 521

## Beschreibung

Die Erfindung betrifft eine Vorrichtung zur biologischen Dekontamination von perkutanen Zugängen oder Stomata von Patienten mit einem Plasmagenerator zur Erzeugung eines dekontaminierenden Plasmas angrenzend an eine Behandlungsfläche de Plasmagenerators.

In der medizinischen Praxis sind für vielfältige Anwendungen verschiedenste Arten von perkutanen ("perkutan" = durch die Haut) Zugängen zu Organen und unterschiedlichen Bereichen im menschlichen Körper erforderlich. Hierzu werden beispielsweise Katheter jeglicher Art (z. B. Blasenkatheter, Ureterkatheter, Portkatheter, Tubenkatheter, Herzkatheter, Dialysekatheter, etc.) sowie vielfältige Arten von äußeren Drainagen (z. B. Redon-Drainage, Kapillar-Drainage, Shirley-Drainage, T-Drainage, Pankreas-Drainage, etc.) eingesetzt, die aus speziellem Schlauchmaterial gefertigt sind und der Ableitung von Körperflüssigkeiten, wie Blut, Gewebsflüssigkeit, Wundsekret und Eiter, wie sie insbesondere nach chirurgischen Eingriffen entstehen können, aus dem Körperinneren nach Außen dienen.

Weiterhin werden für zahlreiche chirurgische Eingriffe ebenfalls chirurgisch angelegte künstliche Öffnungen in der Körperoberfläche (so genannte Stomata) genutzt. Stomata sind z. B. zur Ableitung des Inhalts von Hohlorganen nach Außen (Enterostoma, Colostoma, Ileostoma, Urostoma, Tracheostoma, Gastrostroma), wie beispielsweise im Falle einer künstlichen Darmverlegung (Enterostoma) oder einer künstlichen Harnableitung (Urostoma) etc., bei der Implantation von Endo-Exo-Prothesen, bei denen nach Extremitätenamputation ein knochenverankerter "Endostiel" zur Montage einer äußeren "Exo-Prothese" durch die Haut nach Außen geleitet wird, oder zur Verlegung von perkutanen Steuerkabeln (Drivelines), wie sie beispielsweise zur Verbindung eines implantierten ventrikulären Unterstützungssystems (VAD - ventricular assist device: eine Art "künstliches Herz") mit dem externen Steuergerät genutzt werden, notwendig.

Ein Problem bei der Applikation derartiger Zugänge und Stomata stellt das Infektionsrisiko des betreffenden Hautareals dar. Das Infektionsrisiko in diesem Bereich ist besonders hoch, da einerseits die Haut als größtes Körperorgan, prinzipiell Keimen eine große Angriffsfläche bietet und andererseits Hautkeime durch die Katheter- bzw. Drainage-Eintrittsstellen in den Körper eindringen können, wo sie Komplikationen, wie eine Abszessbildung oder eine lebensbedrohende Sepsis (Blutvergiftung) verursachen können. Daher kommt der Vermeidung bzw. Reduzierung des Infektionsrisikos bei der Verlegung Nachsorge von perkutanen Zugängen und Stomata jeglicher Art eine wesentliche Rolle zu.

Die einen perkutanen Zugang oder ein Stomata umgebenden Hautareale werden hierzu mit einem trocknen, sterilen Verband abgedeckt, regelmäßig inspiziert, gesäubert und antiseptisch behandelt, sowie ggf. prophylaktisch mit Antibiotika behandelt. Ggf. sind darüber hinaus, z.B. bei einer Besiedelung der Haut mit multiresistenten-Erregern, besondere Schutzmaßnahmen, wie beispielsweise eine Dekolonisierung der Haut erforderlich.

Das Restrisiko für Infektionen ist bei perkutanen Zugängen und Stomata hoch, so dass eine weitere Reduzierung des Risikos anzustreben ist. Der Einsatz von Desinfektionsmitteln birgt das Problem von möglichen allergischen Reaktionen. Zudem sind die Desinfektionsmaßnahmen in der Regel zeit- und materialintensiv. Dies gilt insbesondere im Fall von prophylaktischen Maßnahmen gegen eine Infektion durch multi-resistente-Erreger. Zur Förderung einer Wundheilung sind neben den Desinfektionsmaßnahmen zusätzlich noch heilungsfördernde Maßnahmen erforderlich.

Es ist hinreichend bekannt, dass Plasma eine desinfizierende Wirkung hat.

WO 2011/023478 A1 offenbart eine Vorrichtung zur flächigen Behandlung von Bereichen menschlicher oder tierischer Haut- bzw. Schleimhautoberflächen mittels eines kalten Atmosphärendruckplasmas. Hierzu wird eine Manschette mit einer in der Manschette eingebauten Elektrodenanordnung um ein Körperteil gewunden. Der zu behandelnde Bereich ist dabei vollständig durch die Manschette abgedeckt.

Aus WO 2013/167693 A1 ist eine Vorrichtung zur Plasmabehandlung von menschlichen, tierischen oder pflanzlichen Oberflächen, insbesondere von Haut oder Schleimarealen bekannt. Die Vorrichtung hat eine Elektrodenanordnung, bei der die Hochspannungselektrode aus einem dünnen Draht besteht und mit einer Isolationsschicht ummantelt ist. Die geerdete Elektrode besteht aus einer gasdurchlässigen, textilen Flächenstruktur, die sich flexibel auf beliebig gekrümmte Oberflächen auflegen lässt. Ein Gerät nach der Präambel von Anspuch 1 ist aus DE202009011521 bekannt.

Ausgehend hiervon ist es Aufgabe der vorliegenden Erfindung eine Vorrichtung zur biologischen Dekontamination von perkutanen Zugängen oder Stomata vom Patienten zu schaffen.

Die Aufgabe wird durch die Vorrichtung mit den Merkmalen des Anspruchs 1 gelöst.

Vorteilhafte Ausführungsformen sind in den Unteransprüchen beschrieben.

Es wird somit die Verwendung von dekontaminieserendem Plasma zur biologischen Dekontamination von perkutanen Zugängen oder Stomata vorgeschlagen, um dadurch die Infektionsprophylaxe einfacher und effektiver zu gestalten und zudem die Wundheilung zu fördern. Dies gelingt dadurch, dass eine Vorrichtung zur biologischen Dekontamination vom perkutanen Zugängen oder Stomata von Patienten einen Plasmagenerator mit einer Behandlungsfläche hat, die ein dekontaminierendes Plasma bereitstellt, wobei diese Behandlungsfläche zumindest teilweise Umfassen eines perkutanen Zugangs oder Stomata anpasst bei gekrümmt und an den perkutanen Zugang oder Stomata anlegbar ist.

Durch die einen perkutanen Zugang oder Stomata mindestens teilweise umfassende Behandlungsfläche eines Plasmagenerators und durch Anlegen dieser anpassbar gekrümmten Behandlungsfläche an den perkutanen Zugang oder Stomata gelingt es, die Infektionsgefahr durch perkutane Zugänge oder Stomata auf zuverlässige und einfache Weise zu reduzieren. Hierzu kann der Plasmagenerator vorzugsweise zur Erzeugung eines kalten Atmosphärendruckplasmas an der Behandlungsfläche ausgebildet sein. Als Plasmaquellen sind z.B. prinzipiell Plasmajet-Anordnungen, DBE-Anordnung (DBE = dielektrisch behinderte Entladung) oder Korona-Anordnungen geeignet.

Durch die Vorrichtung lässt sich eine deutliche Minimierung des Infektionsrisikos im Bereich von perkutanen Zugängen und Stomata erreichen. Zudem werden allergische Reaktionen vermieden, wie sie ansonsten bei einer Desinfektion von perkutanen Zugängen und Stomata durch Infektionsmittel oftmals auftreten. Weiterhin lassen sich Infektionen durch multi-resistente Erreger bei vergleichsweise geringen Zeit- und Materialaufwand wirksam vermeiden. Die Vorrichtung ermöglicht eine beträchtliche Zeit- und Materialeinsparung bei der Nutzung perkutaner Zugänge oder Stomata im Vergleich zur herkömmlichen Infektionsvorsorge. Weiterhin hat die Vorrichtung eine Förderung der Wundheilung zur Folge, da das zur Dekontamination genutzte Plasma nicht nur eine dekontaminierende, sondern auch eine wundheilungsfördernde Heilung hat.

Ein schlauchförmiger perkutaner Zugang oder ein schlauchförmiges Stomata hat in einer bevorzugten Ausführungsform eine integral hiermit verbundene Behandlungsfläche an der Außenseite eines zum Einleiten oder Ableiten von flüssigkeitgenutzten flexiblen Schlauchs. Damit kann während der Nutzungszeit des perkutanen Zugangs oder des Stomatas permanent oder ohne zusätzliche Installationsarbeit wiederkehrend eine Plasmabehandlung des an die Behandlungsfläche angrenzenden Gewebebereichs erfolgen. Die Vorrichtung ist somit ein perkutaner Zugang oder ein Stomata mit einem schlauchförmigen Gebilde, das eine Plasmabehandlungsfläche hat, die an das schlauchförmige Gebilde anpassbar gekrümmt ist und dieses schlauchförmige Gebildet mindestens teilweise umgibt.

Denkbar ist aber auch eine Ausführungsform, bei der die Behandlungsfläche zylinderförmig ist und der Innenraum der zylinderförmigen Behandlungsfläche zur Aufnahme eines Schlauchs eines perkutanen Zugangs oder Stomata vorgesehen ist. Vor der Nutzung des perkutanen Zugangs oder Stomatas wird somit die Behandlungsfläche des Plasmagenerators auf den Schlauch geschoben, um sodann bei der Anwendung permanente oder in Abständen wiederholt zur Dekontamination aktiviert zu werden.

Denkbar ist aber auch, dass die Vorrichtung zur biologischen Dekontamination von perkutanen Zugängen oder Stomata ausgebildet ist, um temporär zur biologischen Dekontamination eingesetzt zu werden. Bei dieser Ausführungsform hat die Vorrichtung vorzugsweise einen Träger mit einem Handgriff und einer U-förmig gekrümmten Vertiefung. Die Behandlungsfläche des Plasmagenerators liegt dabei an der Innenwand der U-förmig gekrümmten Vertiefung an. Auf diese Weise kann die hierdurch U-förmig gekrümmte Behandlungsfläche angrenzend an dem perkutanen Zugang oder das Stomata im Bereich der Gewebeoberfläche platziert werden, so dass der perkutane Zugang oder das Stomata in die U-förmig gekrümmte Vertiefung eintaucht und an die Behandlungsfläche angrenzt. Dann wird nach Aktivierung des Plasmagenerators durch die Behandlungsfläche ein dekontaminierendes Plasma, wie vorzugsweise kaltes Atmosphärengasplasma, erzeugt und auf diese Weise eine Dekontamination des perkutanen Zugangs oder Stomatas und des angrenzenden Hautbereichs vorgenommen. Dies hat gleichzeitig eine wundfördernde Wirkung auf das Gewebe, welches an den perkutanen Zugang und das Stomata angrenzt.

Eine andere zur temporären Dekontamination geeignete Vorrichtung zeichnet sich vorzugsweise dadurch aus, dass die Vorrichtung eine Zange mit zwei gegeneinander beweglich gelagerten Zangenbacken aufweist. An den einander zugewandten Innenflächen der Zangenbacken ist jeweils eine Behandlungsfläche angeordnet. Auf diese Weise kann die Behandlungsfläche das schlauchförmige Gebilde eines perkutanen Zugangs oder Stomata umgreifen und angrenzend hieran positioniert werden. Mit Hilfe der Zange gelingt es, die Behandlungsfläche an unterschiedliche Durchmesser anzupassen.

Die Integration einer Plasma- Behandlungsfläche eines Plasmagenerators mit den Zangenbacken ermöglicht eine gute Handhabung einer Dekontaminationsvorrichtung, um insbesondere perkutane Zugänge und Stomata möglichst einfach und zuverlässig biologisch zu dekontaminieren.

Für all die genannten Ausführungsformen der Vorrichtung ist es besonders vorteilhaft, wenn die Behandlungsfläche einen flexiblen Träger mit mindestens einer von dem flexiblen Träger umschlossenen Hochspannungselektrode und mit mindestens einer Masseelektrode an der Außenfläche des flexiblen Trägers aufweist. Der flexible Träger ist dabei aus einem geeigneten Isolationsmaterial, wie z.B. ein Dielektrikum ausgebildet und zwischen Hochspannungselektrode und Masselektrode angeordnet. Die Masselektrode befindet sich dabei an der Außenfläche des flexiblen Trägers, die zur Anordnung benachbart an das an dem perkutanen Zugang oder das Stomata vorgesehen ist. Damit wird beim Anlegen einer Hochspannung an die Hochspannungselektrode durch einen Plasmagenerator ein kaltes Atmosphärengasplasma an der die Masseelektrode tragenden Außenfläche des flexiblen Trägers erzeugt. Dieses Plasma kann dann auf das den angrenzenden perkutanen Zugang, ein angrenzendes Stomata und die daran angrenzenden Gewebebereiche eines Patienten wirken. Der Patient kann dabei ein Mensch oder ein Tier sein.

Die Masseelektrode ist eine um den flexiblen Träger gewickelte Drahtwicklung. Die Hochspannungselektrode ist dabei beidseits durch einen flexiblen Träger abgedeckt und somit in den Zwischenraum eines flexiblen Trägers angeordnet. Damit wird bei einer rohrförmigen Behandlungsfläche erreicht, dass sowohl die zylinderförmige Innenfläche, als auch die Außenfläche eine Masseelektrode aufweist. Auf diese Weise wird die Gefahr vor elektrischer Hochspannung reduziert.

Eine mit einer Masseelektrode versehende Außenfläche des flexiblen Trägers kann dann z.B. an eine U-förmig gekrümmte Vertiefung eines Trägers oder an einer Zangenbacke einer Zange angebracht werden, die damit elektrisch geerdet ist.

Zumindest eine Masseelektrode sollte jedenfalls an der zur Anlage an dem perkutanen Zugang oder dem Stomata vorgesehenen Oberfläche der Behandlungsfläche angeordnet sein.

Die Hochspannungselektrode ist besonders vorteilhaft als flexible Drahtelektrode ausgeführt. Sie kann aber auch z.B. aus einem Gitterelement gebildet sein.

Besonders vorteilhaft ist es, wenn der flexible Träger aus einem dielektrischen Kunststoffmaterial, insbesondere aus Polyimid, gebildet ist. Ein solches dielektrisches Kunststoffmaterial eignet sich besonders zur Erzeugung von Plasma mittels Hochspannung. Durch eine besonders hohe, stabile elektrische Spannungsfertigkeit und Plasmabeständigkeit zeichnet sich das Polyimid "Kapton ® CR" der Firma Du Pont aus.

Die Erfindung wird nachfolgend anhand von Ausführungsbeispielen mit den beigefügten Zeichnungen näher erläutert. Es zeigen:
- Figur 1: - Querschnittsansicht einer ersten Ausführungsform einer Vorrichtung zur biologischen Dekontamination von perkutanen Zugängen oder Stomata mit einem schlauchartigen Gebilde, das von einer Plasma- Behandlungsfläche umschlossen ist;
- Figur 2: - Seiten- Teilschnittansicht einer flexibel an einen perkutanen Zugang oder ein Stomata anpassbaren Vorrichtung zur biologischen Dekontamination ;
- Figur 3: - Draufsicht auf eine Ausführungsform einer handgeführten Vorrichtung zur biologischen Dekontamination von perkutanen Zugängen oder Stomata;
- Figur 4: - Skizze einer als Zange ausgeführten Vorrichtung zur biologischen Dekontamination von perkutanen Zugängen oder Stomata;
- Figur 5: - Skizze der in die Zangenbacken der Zange aus Figur 4 integrierbaren Behandlungsfläche.

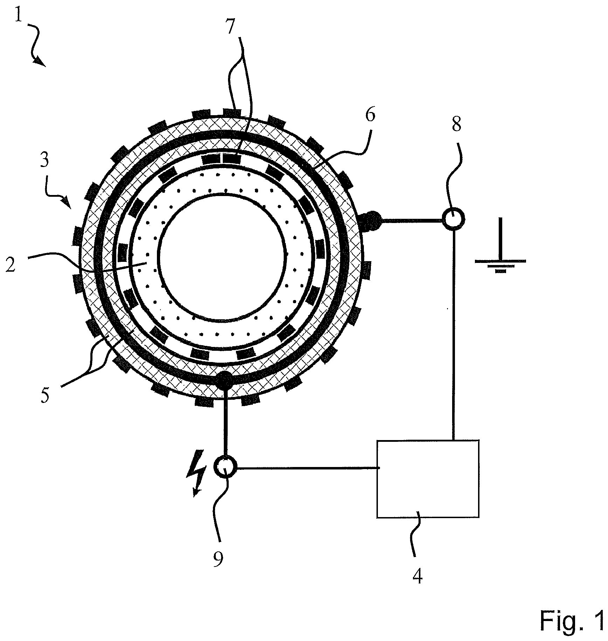

Figur 1 zeigt eine Vorrichtung 1 zur biologischen Dekontamination von perkutanen Zugängen oder Stomata. Ein solcher perkutaner Zugang oder Stomata hat ein schlauchartiges Gebilde 2, an dessen Außenumfang eine Behandlungsfläche eines Plasmagenerators angeordnet ist. Der Plasmagenerator selbst ist ein Generator, der eine Hochspannung erzeugt, wie sie durch den Hochspannungspfeil angedeutet ist.

Die Behandlungsfläche 3 hat einen das schlauchförmige Gebilde 2 umgebenden Isolierstoffmantel 5, in den eine Hochspannungselektrode 6 eingebettet ist. An den beiden Außenseiten des Isolierstoffmantels 5 ist jeweils eine Masseelektrode 7 angeordnet. Diese kann beispielsweise als um den Isolierstoffmantel 5 gewundene Drahtwicklung ausgeführt sein. Denkbar ist aber auch, dass ein Gewebe oder Gitter aus einem elektrisch leitfähigen Material, insbesondere ein Metallgitter, auf der zylinderförmigen Innenfläche und/oder der zylinderförmigen Außenfläche des Isolierstoffmantels 5 angebracht und jeweils mit Massepotential GND verbunden ist.

Hierzu sind ein Masseanschluss 8, der mit der Masseelektrode 7 elektrisch leitend verbunden ist, sowie ein Hochspannungsanschluss 9, der mit der Hochspannungselektrode elektrisch leitend verbunden ist, an die Außenseite der Vorrichtung 1 geführt, um den Hochspannungsgenerator 4 an die Behandlungsfläche 3 anzuschließen.

Durch Beaufschlagung der Hochspannungselektrode 6 mit einer Hochspannung entweder als Gleichspannung, als gepulste Gleichspannung oder Wechselspannung wird in an sich bekannter Weise ein kaltes Atmosphärengasplasma an den Masseelektrodenflächen erzeugt. Dieses Plasma bildet sich dabei in den Luftzwischenräumen der Masseelektroden 7 und tritt dabei an der Oberfläche des schlauchartigen Gebildes 2 auf. Der so erzeugte Plasmastrom gelangt weiterhin an die angrenzenden Gewebeflächen. Auf diese Weise wird eine biologische Dekontamination des schlauchartigen Gebildes 2 des perkutanen Zugangs oder Stomatas und des angrenzendes Gewebes bewirkt. Zudem wird durch das Plasma die Wundheilung des angrenzenden Gewebes verbessert.

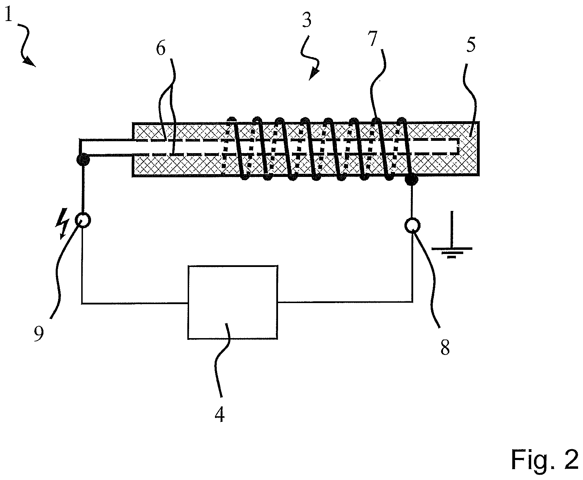

Figur 2 lässt eine perspektivische Seiten-Teilschnittansicht einer Vorrichtung zur Erzeugung einer dielektrisch behinderten Entladung (DBE) mit Hilfe einer Drahtanordnung erkennen. Die Hochspannungselektrode 6 besteht hierbei aus einem Draht, der mit einem geeigneten Isolationsmaterial 5 ummantelt ist. Als geerdete Masseelektrode 7 wird ein Metalldraht verwendet, der spiralförmig den Isolierstoffmantel 5 und die darin eingebettete Hochspannungselektrode 6 umgibt. Werden die Hochspannungselektrode 6 und die Masseelektrode 7 an einen speziellen Hochspannungsgenerator angeschlossen, so wird bei Wahl einer geeigneten Frequenz und Einstellung eines ausreichenden Wertes der Spannung an der Hochspannungselektrode auf der Oberfläche des Isolierstoffmantels 5 im Bereich der spiralförmigen geerdeten Masseelektrode 7 ein Plasma erzeugt. Der Vorteil dieser Elektrodenanordnung besteht vor allem darin, dass sich die Plasmaquelle aufgrund der Flexibilität der Drahtanordnung der Hochspannungselektrode 5 und der Masseelektrode 6 einfach an unterschiedlichste Formen und Größen der perkutanen Zugänge und Stomata anpassen lässt. So kann die Anordnung beispielsweise spiralförmig um ein schlauchartiges Gebilde gewickelt werden. Denkbar ist auch, dass die Vorrichtung 1 teilkreisförmig oder vollkreisförmig geschlossen um die Einführstelle eines perkutanen Zugangs oder Stomatas in einen menschlichen oder tierischen Körper herumgelegt wird, um diesen Wundbereich permanent oder zeitweise zu dekontaminieren.

Wesentlich für die in Figur 2 dargestellte Anordnung ist, dass der Isolierstoffmantel 5, die Hochspannungselektrode 6 und die als Drahtwicklung ausgeführte Masseelektrode 7 zusammen eine flexible an einen gekrümmten perkutanen Zugang oder Stomata anpassbare Anordnung bildet.

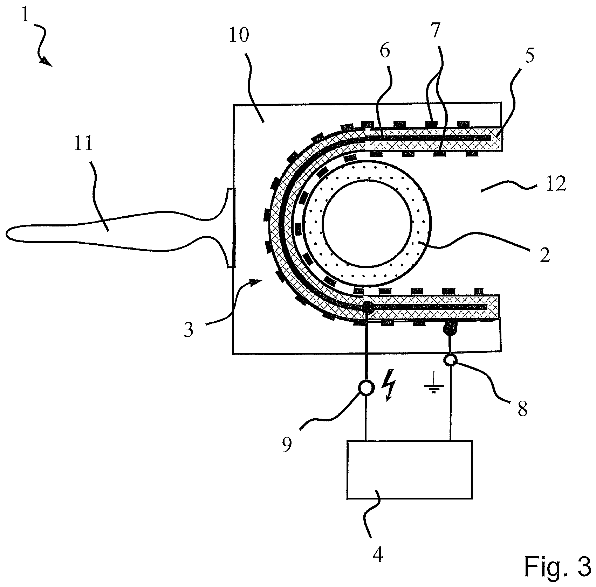

Figur 3 lässt eine Querschnittsanordnung einer anderen Ausführungsform einer Vorrichtung 1 zur biologischen Dekontamination von perkutanen Zugängen in der Draufsicht als Schnittansicht erkennen. Hierbei hat die Vorrichtung 1 einen Träger 10 und einem Handgriff 11 an dem Träger 10. Der Träger 10 hat eine U-förmig gekrümmte Vertiefung 12, in die ein schlauchartiges Gebilde 2 eines perkutanen Zugangs oder Stomata eingeführt werden kann. Die Vorrichtung 1 wird dabei in der Praxis an einem perkutanen Zugang oder Stomata so platziert, dass das schlauchförmige Gebilde 2 in die U-förmige Vertiefung 12 eintaucht. An der Innenseite der U-förmig gekrümmten Vertiefung 12 ist eine Behandlungsfläche 3 des Plasmagenerators 4 angeordnet. Die Behandlungsfläche 3 hat wiederum einen Isolierstoffmantel 5, der eine Hochspannungselektrode 6 elektrisch gegenüber der Masseelektrode 7 isoliert und Spannungsüberschläge verhindert.

Der Isolierstoffkörper 5 mit seiner darin eingebetteten Hochspannungselektrode 6 ist in der U-förmig gekrümmten Vertiefung 12 des Trägers 10 aufgenommen. An der Außenfläche des Isolierstoffkörpers 5, die den nunmehr freien Innenraum der U-förmig gekrümmten Vertiefung 12 zur Aufnahme des schlauchförmigen Gebildes 2 eines perkutanen Zugangs oder Stomatas bildet, ist eine mit Massepotential eines Hochspannungsgenerators 4 verbindbare Masseelektrode 7 angeordnet.

In dem dargestellten Ausführungsbeispiel ist auf der gegenüberliegenden Seite des Isolierstoffmantels 5 angrenzend an den Handgriff 10 ebenfalls eine solche Masseelektrode 7 vorhanden. Damit wird sichergestellt, dass der Träger 10 und der daran angebrachte Handgriff 11 auf jeden Fall mit Massepotential verbunden und geerdet ist. Hierzu kann aber auch der Träger 10 selbst aus einem elektrisch leitenden Material sein und die Masseelektrode 7 bilden.

In dem dargestellten Ausführungsbeispiel ist die Masseelektrode 7 als flexible Drahtelektrode um den Isolierstoffmantel 5 gebunden, so dass die Behandlungsfläche 3 an die gekrümmte U-förmige Vertiefung 12 des Trägers 10 angepasst werden kann.

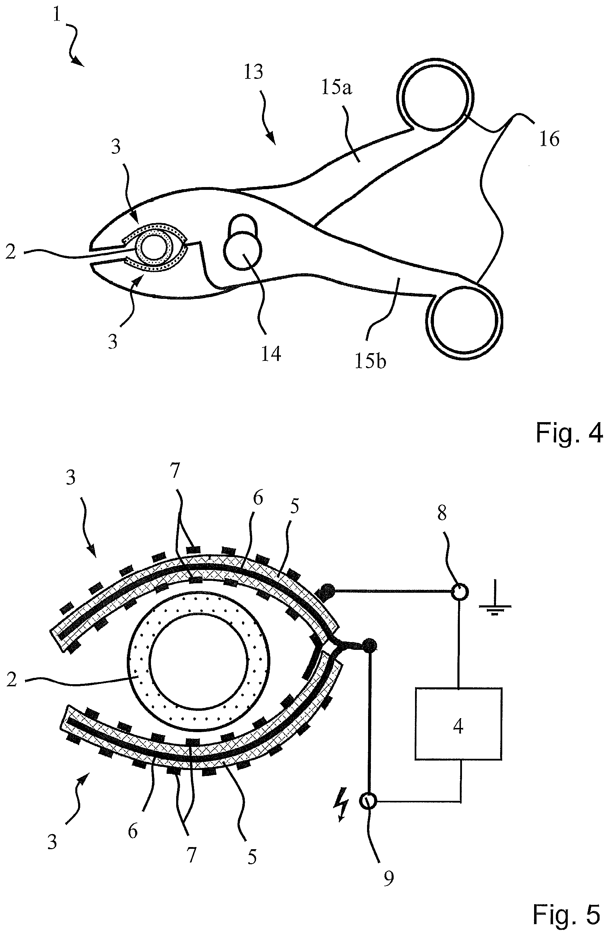

Figur 4 lässt eine weitere Ausführungsform einer Vorrichtung 1 zur biologischen Dekontamination von perkutanen Zugängen oder Stomata erkennen. Diese Vorrichtung hat eine Zange 13 mit zwei relativ zueinander beweglich um ein Schwenklager 14 angeordneten Zangenhebel 15a, 15b, die jeweils an ihrem den Griff 16 gegenüberliegenden Enden eine Zangenbacke 17 haben. Die Zangenbacken 17 sind muldenartig vertieft, so dass sie eine teilkreisförmig oder ovale mindestens teilweise geschlossene Fläche überdecken. In den Zwischenraum zwischen den beiden Zangenbacken 17 kann ein schlauchartiges Gebilde 2 eines perkutanen Zugangs oder Stomatas eingebracht werden. Hierzu werden die Zangenbacken 17 mit Hilfe der Zangenhebel 15a, 15b erst einmal geöffnet, das schlauchartige Gebilde 2 zwischen die Zangenbacken 17 eingeführt und die Zange 13 dann wieder geschlossen.

Deutlich wird, dass an den einander zugewandten Innenflächen der Zangenbacken 17 jeweils eine Behandlungsfläche 3 in der vorher beschriebenen Art angeordnet ist, die mit einem Plasmagenerator 4 (z.B. Hochspannungsgenerator) verbunden wird. Die Behandlungsfläche 3 hat in der vorher beschriebenen Weise einen Isolierstoffmantel 5, der eine Hochspannungselektrode 6 gegenüber einer Masseelektrode 7 abschirmt. Die Masseelektroden 7 der Behandlungsfläche 3 ist an der freien innen liegenden Oberfläche angrenzend an das schlauchartige Gebilde 2 angeordnet. Damit wird z.B. kaltes Atmosphärengasplasma an der freien Innenseite der Behandlungsflächen 3 erzeugt, um auf diese Weise das schlauchartige Gebilde 2 eines perkutanen Zugangs oder Stomata sowie daran anschließendes Gewebe biologisch zu dekontaminieren.

Figur 5 lässt eine vergrößerte Ansicht der Behandlungsflächen 3 für die Vorrichtung 1 aus Figur 4 in der Schnittdarstellung erkennen. Deutlich wird, dass die Behandlungsflächen 3 an die muldenartige Vertiefung der Zangenbacken 17 gekrümmt angepasst sind. Hierzu sind die Behandlungsflächen 13 vorzugsweise aus eine flexiblen Träger in Form des Isolierstoffmantels 5 gebildet. Der Isolierstoffmantel 5 nimmt, wie bei den vorhergehenden Ausführungsformen die ebenfalls angepasst gekrümmte Hochspannungselektrode 6 auf, die als gebogene Platte oder als Stab ausgeführt sein kann. Mindestens an der Innenseite des Isolierstoffmantels 5 ist die Masseelektrode 7 angeordnet, welche mit dem Masseanschluss des Hochspannungsgenerators 4 verbindbar ist. Die Hochspannungselektroden 6 der einander gegenüberliegenden Behandlungsflächen 3 sind ihrerseits mit dem Hochspannungsanschluss des Hochspannungsgenerators 4 (Plasmagenerators) verbunden.

In der dargestellten Ausführungsform sind die Masseelektroden 7 wiederum als gewickelte Drahtelektroden ausgeführt, die beidseits den Isolierstoffmantel umgeben und sowohl an der Innenseite, als auch an der Außenseite, die an dem Träger 10 anliegt, auf der Oberseite des Isolierstoffmantels 5 aufliegen.

Weitere Formen dieser Vorrichtung sind denkbar. Neben diesen dargestellten Drahtanordnungen in O-form, U-form oder Zangenform sind auch spiralförmige, stiftförmige oder ähnliche Ausführungsformen der Behandlungsfläche denkbar. Möglich sind auch Behandlungsflächen, die ein schlauchförmiges Gebilde eines perkutanen Zugangs oder Stomatas mindestens teilweise umschließen oder z.B. mit einer Zange temporär an dem Außenumfang eines perkutanen Zugangs oder Stomatas platziert werden können, wobei von einem Plasmagenerator erzeugtes Plasma als Gasstrom oder Fluidstrom durch Hohlräume der Behandlungsfläche zu dem perkutanen Zugang oder Stomata geleitet werden. Bei dieser Ausführungsform wird das zur biologischen Dekontaminierung verwendete Plasma somit nicht direkt an der Behandlungsfläche erzeugt, sondern in dem Plasmagenerator. Von dort wird es zur Behandlungsfläche geleitet.

Im Sinne der vorliegenden Erfindung kann eine Behandlungsfläche somit eine passive Behandlungsfläche zum Verteilen von in einem Plasmagenerator erzeugten Plasma oder eine aktive Behandlungsfläche zum Erzeugen und flächigem Verbreiten von Plasma sein.

Eine biologische Dekontamination von perkutanen Zugängen oder Stomata von menschlichen oder tierischen Patienten erfolgt mit einer seiner oben beschriebenen Vorrichtung 1 durch die Verfahrensschritte
- mindestens teilweises Umfassen des perkutanen Zugangs oder Stomatas mit einer gekrümmten Behandlungsfläche eines Plasmagenerators;
- Anlegen der Behandlungsfläche an dem perkutanen Zugang oder der Stomata und
- Erzeugen von dekontaminierendem Plasma an der am perkutanen Zugang oder dem Stomata anliegenden Behandlungsfläche.

Unter "Anlegen" wird verstanden, dass die Behandlungsfläche direkt an einem schlauchförmigen Gebilde 2 des perkutanen Zugangs oder Stomatas anliegt oder unmittelbar beabstandet zu dem schlauchförmigen Gebilde 2 mit einem Zwischenraum angrenzend angeordnet ist. Es ist somit nicht erforderlich, dass die Behandlungsfläche 3 dem perkutanen Zugang oder das Stomata zwingend berühren muss. Es muss nur derart anliegen, dass an der Behandlungsfläche 3 vorhandenes Plasma dekontaminierend wirksam mit der Oberfläche des perkutanen Zugangs oder des Stomatas und ggf. des daran angrenzenden Gewebes in Verbindung tritt.

## Patentansprüche

1. Vorrichtung (1) zur biologischen Dekontamination von perkutanen Zugängen oder Stomata von Patienten mit einem Plasmagenerator (4) zur Erzeugung eines dekontaminierenden Plasmas angrenzend an eine Behandlungsfläche (3) des Plasmagenerators (4), wobei die Behandlungsfläche (3) zum mindestens teilweisen Umfassen eines perkutanen Zugangs oder Stomata anpassbar gekrümmt und an den perkutanen Zugang oder das Stomata anlegbar ist, wobei die Behandlungsfläche (3) einen flexiblen Träger (5) mit mindestens einer von dem flexiblen Träger (5) umschlossenen Hochspannungselektrode (6) und mit mindestens einer Masseelektrode (7) an der Außenfläche des flexiblen Trägers (5) hat,
**dadurch gekennzeichnet, dass**
die Masseelektrode (7) eine um den flexiblen Träger (5) gewickelte Drahtwicklung ist.

2. Vorrichtung (1) nach Anspruch 1, **dadurch gekennzeichnet, dass** die Vorrichtung einen Träger (10) mit einem Handgriff (11) und einer U-förmig gekrümmten Vertiefung (12) hat, und dass die Behandlungsfläche (3) des Plasmagenerators (4) an der Innenwand der U-förmig gekrümmten Vertiefung (12) anliegt.

3. Vorrichtung (1) nach Anspruch 1, **dadurch gekennzeichnet, dass** die Behandlungsfläche (3) zylinderförmig ist und der Innenraum der zylinderförmigen Behandlungsfläche (3) zur Aufnahme eines Schlauchs (2) eines perkutanen Zugangs vorgesehen ist.

4. Vorrichtung (1) nach Anspruch 1, **dadurch gekennzeichnet, dass** die Vorrichtung (1) eine Zange (13) mit zwei gegeneinander beweglich gelagerten Zangenbacken (17) hat, und dass jeweils eine Behandlungsfläche (3) an den einander zugewandten Innenflächen der Zangenbacken (17) angeordnet ist.

5. Vorrichtung nach Anspruch 1, **dadurch gekennzeichnet, dass** die Masseelektrode (7) an der zur Anlage an den perkutanen Zugang oder dem Stomata vorgesehenen Außenseite der Behandlungsfläche (3) angeordnet ist.

6. Vorrichtung (1) nach einem der Ansprüche 1 oder 5, **dadurch gekennzeichnet, dass** die Hochspannungselektrode (6) eine flexible Drahtelektrode ist.

7. Vorrichtung (1) nach einem der vorhergehenden Ansprüche, **dadurch gekennzeichnet, dass** der flexible Träger (5) aus einem dielektrischen Kunststoffmaterial, insbesondere Polyimid, gebildet ist.

8. Vorrichtung (1) nach einem der vorhergehenden Ansprüche, **dadurch gekennzeichnet, dass** der Plasmagenerator (4) mit seiner mindestens einen Behandlungsfläche (3) zur Erzeugung von kaltem Atmosphärendruckplasma eingerichtet ist.

9. Verwendung einer Vorrichtung (1) nach einem Ansprüche 1 bis 8 zur biologischen Dekontamination von perkutanen Zugängen oder Stomata von Patienten.

## Claims

1. A device (1) for biologically decontaminating percutaneous access points or stomata of patients, including a plasma generator (4) for generating a decontaminating plasma adjacent to a treatment surface (3) of the plasma generator (4), wherein the treatment surface (3) is adaptably curved to at least partially enclosing a percutaneous access point or stoma and is placeable so as to rest at the access point or the stoma, wherein the treatment surface (3) has a flexible support (5) with at least one high voltage electrode (6) encompassed by the flexible support (5) and with at least one ground electrode (7) at the outer surface of the flexible support (5),
**characterized in that**
the ground electrode (7) is a wire winding wound around the flexible support (5).

2. The device (1) according to Claim 1, **characterized in that** the device has a support (10) with a handle (11) and a U-shapedly curved recess (12), and **in that** the treatment surface (3) of the plasma generator (4) rests at the inner wall of the U-shapedly curved recess (12).

3. The device (1) according to Claim 1, **characterized in that** the treatment surface (3) is cylinder-shaped and the inside of the cylinder-shaped treatment surface (3) is provided for receiving a tube (2) of a percutaneous access point.

4. The device (1) according to Claim 1, **characterized in that** the device (1) has forceps (13) with two forceps jaws (17) movably mounted relative to each other and **in that** one treatment surface (3) is arranged on each of the inner surfaces of the forceps jaws (17) facing each other.

5. The device according to Claim 1, **characterized in that** the ground electrode (7) is arranged on the outer side of the treatment surface (3) which is provided to be placed so as to rest at the percutaneous access point or the stoma.

6. The device (1) according to any one of Claims 1 or 5, **characterized in that** the high voltage electrode (6) is a flexible wire electrode.

7. The device (1) according to any one of the preceding claims, **characterized in that** the flexible support (5) is formed from a dielectric plastics material, in particular polyimide.

8. The device (1) according to any one of the preceding claims, **characterized in that** the plasma generator (4) is configured with its at least one treatment surface (3) to generate cold atmospheric pressure plasma.

9. Use of a device (1) according to any one of Claims 1 to 8 for biologically decontaminating percutaneous access points or stomata of patients.

## Revendications

1. Dispositif (1) pour la décontamination biologique d'accès percutanés ou de stomates de patients avec un générateur de plasma (4) pour produire un plasma décontaminant à proximité d'une surface de traitement (3) du générateur de plasma (4), dans lequel la surface de traitement (3) est adaptable sous une forme incurvée pour recouvrir au moins en partie un accès percutané ou un stomate et peut reposer sur l'accès percutané ou le stomate, dans lequel la surface de traitement (3) possède au moins un support flexible (5) avec au moins une électrode à haute tension (6) entourée par le support flexible (5) et avec au moins une électrode de masse (7) à la surface extérieure du support flexible (5),
**caractérisé en ce que**
l'électrode de masse (7) est un bobinage de fil enroulé autour du support flexible (5).

2. Dispositif (1) selon la revendication 1, **caractérisé en ce que** le dispositif possède un support (10) avec une poignée (11) et une cavité (12) incurvée en forme de U, et que la surface de traitement (3) du générateur de plasma (4) repose sur la paroi intérieure de la cavité (12) incurvée en forme de U.

3. Dispositif (1) selon la revendication 1, **caractérisé en ce que** la surface de traitement (3) est cylindrique et l'espace intérieur de la surface de traitement cylindrique (3) est prévu pour recevoir un tuyau (2) d'un accès percutané.

4. Dispositif (1) selon la revendication 1, **caractérisé en ce que** le dispositif (1) possède une pince (13) avec deux becs de pince (17) montés de façon mobile l'un par rapport à l'autre et qu'une surface de traitement (3) est disposée respectivement sur les faces intérieures des becs de pince (17) tournées l'une vers l'autre.

5. Dispositif (1) selon la revendication 1, **caractérisé en ce que** l'électrode de masse (7) est disposée sur le côté extérieur de la surface de traitement (3) prévu pour reposer sur l'accès percutané ou le stomate.

6. Dispositif (1) selon l'une quelconques des revendications 1 ou 5, **caractérisé en ce que** l'électrode à haute tension (6) est un fil-électrode flexible.

7. Dispositif (1) selon l'une quelconque des revendications précédentes, **caractérisé en ce que** le support flexible (5) est constitué d'une matière plastique diélectrique, en particulier de polyimide.

8. Dispositif (1) selon l'une quelconque des revendications précédentes, **caractérisé en ce que** le générateur de plasma (4) est ajusté avec au moins une surface de traitement (3) pour produire un plasma froid à pression atmosphérique.

9. Utilisation d'un dispositif (1) selon l'une quelconque des revendications 1 à 8 pour la décontamination biologique d'accès percutanés ou de stomates de patients.
